# EUROPEAN PATENT SPECIFICATION

(11) **EP 0 743 933 B1**
(45) Date of publication and mention of the grant of the patent: **09.06.1999**
(21) Application number: 95910142.9
(22) Date of filing: 06.02.1995
(51) Int. Cl.: C07C 17/386, C07C 19/08

(54) **PROCESS FOR SEPARATING PENTAFLUOROETHANE FROM A MIXTURE COMPRISING HALOGENATED HYDROCARBONS AND CHLOROPENTAFLUOROETHANE**
VERFAHREN ZUR TRENNUNG VON PENTAFLUORETHAN AUS EINER MISCHUNG VON HALOGENIERTEN KOHLENWASSERSTOFFEN UND CHLOROPENTAFLUORETHAN
PROCEDE DE SEPARATION DU PENTAFLUOROETHANE D'UN MELANGE D'HYDROCARBURES HALOGENES ET DE CHLOROPENTAFLUOROETHANE

(30) Priority: 07.02.1994 US 192663
(43) Date of publication of application: 27.11.1996
(73) Proprietor: E.I. DU PONT DE NEMOURS AND COMPANY, Wilmington Delaware 19898 (US)
(72) Inventor: MAHLER, Barry, Asher, Glen Mills, PA 19342-1258 (US); MILLER, Ralph, Newton, Newark, DE 19711-2477 (US)
(74) Representative: BROOKES & MARTIN
(86) International application number: US9501186
(87) International publication number: WO9521147

(56) References cited:
- EP-A- 0 472 391
- EP-A- 0 564 036
- EP-A- 0 626 362
- US-A- 5 087 329
- RESEARCH DISCLOSURE, vol. 360, 1994 EMSWORTH GB, pages 191-193, 'Methods for Separating Chloro-Carbons from Hydrofluoroalkanes'

## Description

The instant invention relates to a process for separating pentafluoroethane from a mixture comprising pentafluoroethane and chloropentafluoroethane by extractive distillation employing hydrocarbon, hydrochlorocarbon or chlorocarbon extractive agents.

### BACKGROUND OF THE INVENTION

New regulations have been designed to protect the stratospheric ozone layer from possible damage by fully halogenated chlorofluorocarbons. Pentafluoroethane (CHF2CF3 or HFC-125) is a valuable non-chlorine containing fluorocarbon that is especially valuable as a refrigerant, blowing agent, propellant, fire extinguishing agent, sterilant carrier gas, among other uses.

Pentafluoroethane may be prepared by chlorofluorinating perchloroethylene (perclene or CCl₂CCl₂) to produce a mixture comprising trichlorotrifluoroethane (CF2ClCFCL2 or CFC-113), dichlorotetrafluoroethane (CF2ClCF2Cl or CFC-114) and dichlorotrifluoroethane (CFCl2CHF2 or HCFC-123), and, after removing trichlorotrifluoroethane, fluorinating the remaining mixture to produce a mixture comprising pentafluoroethane (HFC-125), chloropentafluoroethane (CF3CF2Cl or CFC-115), and smaller amounts of other fluorinated compounds, e.g., hexafluoroethane (CF3CF3 or FC-116). Such a chlorofluorinating method is described in U.S. Patent No. 3,755,477. Various other methods for making pentafluoroethane together with chloropentafluoroethane are known and are described, for example, in U.S. Patent Nos. 3,518,500, 3,258,500, Japanese Patent Application Publication Nos. JP 03/099026, JP 04/029941, European Patent Application Publication No 0 506 525, and World Intellectual Property Organization Publication No. WO 91/05752. The disclosure of the previously identified patent documents is hereby incorporated by reference.

Whenever an extractive distillation process is employed for separating certain compounds, it is necessary to discover an extractive agent which will be effective to sufficiently enhance the desired separation process. Methods which have been used to predict what extractive agents are likely to work have been described by L. Berg in Chem. Eng. Progress, Vol. 65, No. 9, pages 52-57, Sept. 1969; the disclosure of which is hereby incorporated by reference. When discussing extractive distillation, it was stated in the Berg article that "hydrogen bonds appear to be an important factor since all successful extractive distillation agents are highly hydrogen bonded liquids ..... Thus, the criteria for successful extractive agents are that they boil considerably higher than the compounds being separated, form no minimum azeotropes with the components, and be a highly hydrogen bonded liquid, that is, Class I or Class II of the hydrogen bond classification. Phenols, aromatic amines (aniline and its derivatives), higher alcohols, glycols, etc. are examples of successful extractive agents."

Extractive distillation agents that are capable of separating pentafluoroethane from chloropentafluoroethane are disclosed in US Patent No. 5,087,329 in which chlorofluorocarbons such as dichlorotetrafluoroethane (CFC-114) are employed. However, these CFC compounds are relatively expensive, and regulations concerning protection of the ozone layer will likely cause CFC compounds to be phased out as commercial products thereby making CFCs unavailable or uneconomic to use. The disclosure of US Patent No. 5,087,329 is hereby incorporated by reference.

EP-A-0626362 discloses a purification process for purifying pentafluoroethane (HFC-125) by extractive distillation of crude HFC-125 to separate HFC-125 from byproduct monochloropentafluoroethane (CFC-115). The extractive reagents which are disclosed in this document and which are also disclosed in the Japanese priority document are limited to n-hexane, diethyl ether, ethyl acetate, acetone and methyl ethyl ketone.

### SUMMARY OF THE INVENTION

Conventionally used method for manufacturing pentafluoroethane typically produce a mixture comprising pentafluoroethane (HFC-125) and chloropentafluoroethane (CFC-115). Such a mixture can form an azeotropic or azeotrope-like composition. The boiling points of pentafluoroethane and chloropentafluoroethane are relatively close, i.e., about -48.5 degrees C for pentafluoroethane and about -38.7 degrees C for chloropentafluoroethane, and their relatively volatility is below about 1.1 at concentrations of pentafluoroethane greater than about 87.5 mole%, and below about 1.01 at concentrations greater than about 95 mole%. The above boiling points and relative volatilities indicate that it would be extremely difficult, if not impossible, to recover substantially pure pentafluoroethane from such a mixture by conventional distillation. By "conventional distillation", it is meant that the relative volatility only of the components of the mixture to be separated is being used to separate certain compounds. It was, therefore, surprising and unexpected that the instant invention can successfully employ not highly hydrogen bonded compounds as extractive distillation agents for separating pentafluoroethane from chloropentafluoroethane. By "extractive distillation" it is meant that a third component is added to the mixture to be separated so as to modify the relative volatilities of the compounds to be separated. By "not highly hydrogen bonded" it is meant that the extractive agent(s) are not characterized as being Class I or Class II of the hydrogen bond classification. The extractive distillation agents of the invention are also relatively inexpensive and readily available.

According to the present invention there is provided a process for separating chloropentachloroethane from a first mixture comprising chloropentafluoroethane and pentafluoroethane, the process comprising the steps of:
adding at least one extractive agent to the first mixture in order to form a second mixture, wherein the extractive agent is fluorine free and does not hydrogen bond; and wherein the extractive agent has an atmospheric boiling point greater than 28 degrees C, but excluding the use of an extractive agent consisting of n-hexane, diethylether, ethyl acetate, acetone or methylethyl ketone,
separating the chloropentafluoroethane from the pentafluoroethane of the second mixture by extractively distilling the second mixture in an extractive distillation zone, and;
recovering pentafluoroethane.

The instant invention solves the problems associated with conventional distillation methods by employing an extractive distillation process for separating pentafluoroethane from a first mixture comprising pentafluoroethane, chloropentafluoroethane and optionally hydrochlorofluorocarbons (HCFCs) such as HCFC-124, 124a or hydrofluorocarbons such as HFC-143a, among others, which comprise:
(i) adding an extractant agent comprising at least one of a hydrocarbon as defined above such as pentane, heptane, octane, among others, and/or a hydrochlorocarbon such as triclene (CHClCCl₂), and/or a chlorocarbon such as perclene (CCl₂CCl₂), among others to the first mixture in order to form a second mixture; wherein the extractant is a non-hydrogen bonder having two or more carbon atoms and an atmospheric boiling point greater than about 28 degrees C and less than about 126 degrees C,
(ii) separating pentafluoroethane from chloropentafluoroethane of the second mixture by extractively distilling the second mixture in an extractive distillation zone and thereby recovering as overhead product, a pentafluoroethane stream substantially free of chloropentafluoroethane.

The first mixture can be obtained from any suitable manufacturing process of source. For example, the first mixture can be obtained as product stream from the process disclosed in WIPO Publication No. WO 92/19576, the disclosure of which is hereby incorporated by reference.

The extractive agents used in the present invention are available from a number of suppliers at relatively low cost, and are much less expensive than fluorine-containing extractive agents, e.g. CFCs. The extractive agents of the instant invention can also be readily removed, for example, from the stream that exits from the bottom of the distillation column. The extractive agents can be removed from the steam containing chloropentafluoroethane by simple distillation, and, if desired, reintroduced into the separation System.

Hydrocarbons that can be used as extractive agents are fluorine-free and comprise 5 to 8 carbon atoms. Specific examples of such hydrocarbons comprise or consist essentially of at least one member from group of normal or n-pentane, heptane, octane, their isomers and/or cyclic compounds thereof, among others, that possess an atmospheric boiling point from about 28 to about 126 C; normally about 28 to about 60°C. While hydrocarbons with higher boiling points and with more than 8 carbon atoms are also effective, such hydrocarbons are less desirable because they will typically require higher energy consumption, e.g., higher heat input.

Chlorocarbons (CCs) and hydrochlorocarbons (HCCs) that can be used as extractive agents comprise 2 carbon atoms and possess an atmospheric boiling point from about 74 to about 121° C. Specific examples comprise or consist essentially of at least one member from the group of perchloroethylene (perclene), methyl chloroform, 1,1 dichloroethane, 1,2 dichloroethane, trichloroethylene, ethylene dichloride, isomers and cyclic compounds thereof, among others. Higher boiling chlorocarbons and hydrochlorocarbons and those containing more than 2 carbon atoms are also effective, such compounds are less desirable because they will normally require higher energy consumption.

In one aspect of the invention, mixtures comprising or consisting essentially of the previously identified hydrocarbons, HCCs, and/or chlorocarbons can be employed as the extractive agent so long as the mixture has an atmospheric boiling point greater than about 28 degrees C.

### BRIEF DESCRIPTION OF THE DRAWING

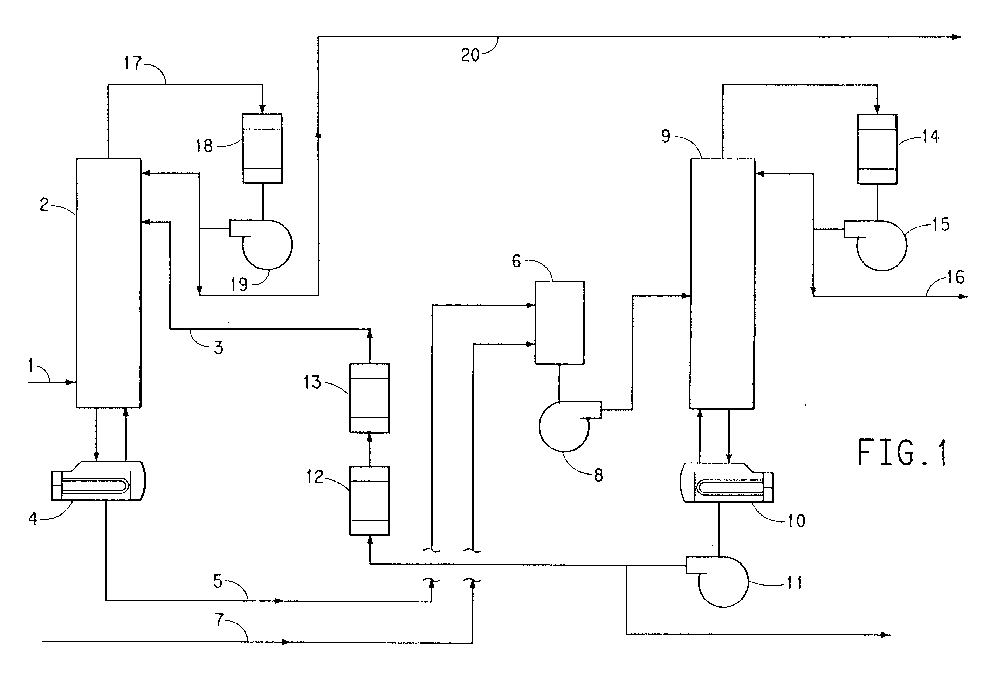

Figure 1-Fig. 1 is a schematic diagram of an extractive distillation system which can be used for practicing the process of the invention.

### DETAILED DESCRIPTION

The pentafluoroethane (HFC-125) and chloropentafluoroethane (CFC-115), which are the primary constituents of a first mixture, in their separated and pure state have boiling points, respectively, of about 48.5 and -38.7 degrees C. The relative volatility at atmospheric pressure of pentafluoroethane/chloropentafluoroethane was found to be nearly 1.0 as 100% purity of pentafluoroethane was approached. These data indicate that using conventional distillation procedures will not result in the separation of a substantially pure compound.

To determine the relative volatility of pentafluoroethane and chloropentafluoroethane the so-called PTx Method is used. In this procedure, the total absolute pressure in a cell of known volume is measured at a constant temperature for various known binary compositions. Use of the PTx Method is described in greater detail in "Phase Equilibrium in Process Design", Wiley-Interscience Publisher, 1970, written by Harold R. Null, on pages 124 to 126; the entire disclosure of which is hereby incorporated by reference.

These measurements can be correlated to equilibrium vapor and liquid compositions in the cell by using an activity coefficient equation model, such as the Non-Random, Two-Liquid (NRTL) equation, to represent liquid phase non-idealities. Use of an activity coefficient equation, such as the NRTL equation, is described in greater detail in "The Properties of Gases and Liquids, 4th edition, publisher McGraw Hill, written by Reid, Prausnitz and Poling, on pages 241 to 387; and in "Phase Equilibria in Chemical Engineering, published by Butterworth Publishers, 1985, written by Stanley M. Walas, pages 165 to 244; the entire disclosure of each of the previously identified references is hereby incorporated by reference.

The result of PTx measurements and the above series of calculations for a binary mixture containing HFC-125 and CFC-115 are summarized in Tables 1 through 3, giving results for -25 degrees C, 0 degrees C and 25 degrees C. Similar measurements were obtained and similar calculations were performed for the other binary combinations of compounds of the mixtures in the invention.

Without wishing to be bound by any theory or explanation, it is believed that the NRTL equation can sufficiently predict whether or not pentafluoroethane and chloropentafluoroethane mixtures and/or the following other mixtures behave in an ideal manner, and can sufficiently predict the relative volatilities of the components in such mixtures.

**Table 1**

| Vapor-Liquid Measurements on the HFC-125/CFC-115 System at -25 deg. C | | | | |
|---|---|---|---|---|
| Mole % CFC-115 | | | Pressure Meas. (psia) | Relative Volatility HFC-125/CFC-115 |
| Charge | Liquid | Vapor | | |
| 17.67 | 17.72 | 15.85 | 40.75 | 1.144 |
| 13.48 | 13.51 | 12.38 | 40.75 | 1.105 |
| 10.40 | 10.42 | 9.74 | 40.95 | 1.078 |
| 7.40 | 7.42 | 7.08 | 41.10 | 1.052 |

**Table 2**

| Vapor-Liquid Measurements on the HFC-125/CFC-115 System at 0 deg. C | | | | |
|---|---|---|---|---|
| Mole % CFC-115 | | | Pressure Meas. (psia) | Relative Volatility HFC-125/CFC-115 |
| Charge | Liquid | Vapor | | |
| 17.67 | 17.78 | 15.92 | 96.95 | 1.142 |
| 13.48 | 13.55 | 12.37 | 97.20 | 1.110 |
| 10.40 | 10.45 | 9.69 | 98.00 | 1.087 |
| 7.40 | 7.43 | 7.00 | 98.00 | 1.066 |

**Table 3**

| Vapor-Liquid Measurements on the HFC-125/CFC-115 System at 25 deg. C | | | | |
|---|---|---|---|---|
| Mole % CFC-115 | | | Pressure Meas. (psia) | Relative Volatility HFC-125/CFC-115 |
| Charge | Liquid | Vapor | | |
| 17.67 | 17.87 | 16.36 | 198.60 | 1.113 |
| 13.48 | 13.61 | 12.65 | 200.25 | 1.087 |
| 10.40 | 10.49 | 9.88 | 200.75 | 1.069 |
| 7.40 | 7.45 | 7.11 | 201.00 | 1.052 |

The "Charge" column refers to the quantity of CFC-115 that is in a mixture of HFC-125 and CFC-115 that was introduced into the PTx cell. The "Relative/Volatility" and the Mole % CFC-115 in the Vapor and Liquid were those calculated from the Pressure measured at the temperature indicated on the above Tables.

While the relative volatility of CFC-115 in comparison to HFC-125 at relatively low concentrations is sufficient to permit separating CFC-115 by using conventional distillation methods, the relative volatility is nearly 1.0 as 100% purity of HFC-125 as approached. A relative volatility approaching 1.0 would render removal of low concentrations of CFC-115 from HFC-125 by using conventional distillation difficult if not impossible, e.g., conventional distillation would require using large and expensive distillation columns.

Using the equations developed for the above system at temperatures of about -25, 0 and about 25 degrees C, and when extrapolating to about -50 degrees C and a pressure of about 10 to about 20 psia, the presence of a low-boiling azeotropic or azeotrope-like mixture is indicated, with a composition of about 10 mole % CFC-115 and 90 mole % HFC-125.

For best results, the inventive process herein avoids using conditions that cause formation of the HFC-125/CFC-115 azeotropic or azeotrope-like compositions.

The problems associated with conventional distillation methods are overcome in the present invention by using an extractive distillation method. Extractive distillation depends upon the ability of certain extractive agents to increase the relative volatility of the binary pair of compounds to be separated. Extractive distillation is typically performed by operating a continuous distillation column, which comprises a multi-stage distillation column, with a minimum of two feed points, e.g. introducing the extractive agent at a first feed point which is located above the second feed point that is used for introducing the mixture to be separated, a reboiler and an overhead condenser for returning reflux to the column.

Given the relative volatilities of pentafluoroethane and chloropentafluoroethane, it was a surprising and an unexpected result that they can be purified by extractive distillation using certain readily available and low cost non-fluorine containing compounds that are not highly hydrogen bonded and, in some cases, fluorine-free, e.g., hydrocarbons. The invention process can produce a purified HFC-125 product that is substantially free of CFC-115. By "substantially free" it is meant that the HFC-125 contains less than about 1.0 wt. % CFC-115, normally less than about .05 wt%, and in some cases less than about 10 ppm by weight CFC-115.

In one aspect of the invention, an extractive agent, e.g. normal heptane, is introduced at an upper feed point of the distillation column, whereas the mixture requiring separation, e.g. pentafluoroethane and chloropentafluoroethane, is introduced at a relatively lower point in the column. The liquid extractive agent passes downwardly through trays which are located in the center of the column. While in the presence of the extractive agent, pentafluoroethane is relatively more volatile than chloropentafluoroethane, thereby allowing pentafluoroethane which is substantially free of chloropentafluoroethane to exit the top of the column, where the pentafluoroethane many be condensed by using conventional reflux condensers. At least a portion of this condensed stream can be returned to the top of the column as reflux, and the remainder recovered as a useful product. If desired the exiting pentafluoroethane can be recycled to the column or a second column for further purification.

The chloropentafluoroethane and an extractive agent exiting the bottom of the column can then be passed to a stripper or distillation column for separation by using conventional distillation or other known methods. If desired, the extractive agent may then be recycled to the extractive distillation column. In some cases, the CFC-115 can be separated from the remaining constituents of the stream by using conventional distillation or other known methods.

The ratio of the material exiting the top of the column, which is then condensed and in turn returned to the column, to the amount of material which is removed as product is commonly referred to as the reflux ratio. The reflux ratio will define the physical characteristics of the distillation column. In general, an increase in the reflux ratio will in turn cause an increase in the purity of the recovered HFC-125, e.g., the quantity of extractant in the recovered HFC-125 can be reduced. Increasing the reflux ratio typically requires employing larger and more expensive distillation column.

The specific conditions which can be used for practicing the invention are interrelated and depend upon a number of design parameters such as the diameter of the column, selected feed points, the number of separation stages in the column, reflux ratio, among others. The operating pressure of the distillation system may range from about 15 to 350 psia, normally about 50 to 300 psia. Typically, an increase in the extractant feed rate relative to the mixture to be separated will result in an increase in the purity of the pentafluoroethane produced. The temperature and heat transfer area of the overhead condenser is normally sufficient to substantially fully condense the pentafluoroethane, or is optionally sufficient to achieve the desired reflux ratio by partial condensation.

The temperature that is employed at a given step in the inventive process is a function of the pressure and the design characteristics of the distillation column, e.g., the ratio of extractive agent to the first mixture. Typically, the temperature will range from about -25 to 200° C based upon an operating pressure of about 200 psia.

The quantity of CFC-115 in the first mixture, i.e., the HFC-125-containing mixture to be purified, can be reduced by using conventional distillation. While conventional distillation methods are incapable of producing HFC-125 that is substantially free of CFC-115, conventional distillation can be used for reducing the initial quantity of CFC-115 in the first mixture. For example, conventional distillation can be used for removing relatively large or bulk quantities of CFC-115 from HFC-125, which in turn is processed in accordance with the invention method, in order to obtain a substantially CFC-115-free HFC-125 product. Depending upon the quantity of HFC-125 in the first mixture, HFC-125 can be removed from a mixture containing relatively large quantities of CFC-115 thereby producing CFC-115 that is substantially free of HFC-125.

The effective amount of the extractive agent(s) can vary widely. In general, however, using an increased amount of extractive agent will enhance the purity of the recovered HFC-125, e.g., the removal rate of CFC-115 is increased. Typically, the ratio of extractive agent to HFC-125 ranges from about 2 to about 3:1 on a weight basis. But higher ratios may be necessary for the more difficult separations.

Referring now to the Figure, Fig. 1 schematically illustrates a system which can be used for operating the inventive distillation process. A first mixture comprising pentafluoroethane and chloropentafluoroethane is supplied via conduit 1 to extraction column 2. At least one liquid extractive agent is supplied via conduit 3 to the extraction column 2, and introduced into column 2 at a location above the mixture 1. A second mixture comprising extractive agent(s), chloropentafluoroethane and other impurities, is removed from the bottom of column 2 and transported to steam-heated reboiler 4. In some cases, the reboiler 4 is attached to the extractive column 2. The second mixture is supplied via conduit 5 to a feed tank 6. Supplemental liquid extractive agent is also supplied to feed tank 6 via conduit 7 thereby forming a third mixture or extraction agent recycle. A pump 8 transports the third mixture to a stripping column 9. Stripping column 9 separates the extractive agent from non-extractive agents. Extractive agent is removed from column 9 and supplied to a second steam heated reboiler 10. In some cases, the reboiler 10 is attached to column 9. Pump 11 transports the extractive agent from the reboiler 10 through a cold water chiller 12, and then to chiller 13. If necessary, excess quantities of extraction agent can be purged prior to reaching chiller 12. Typically, chiller 13 is operated at a temperature of about -25° C. After exiting chiller 13, the extraction agent is supplied via conduit 3 into extraction column 2.

CFC-115 exits from the top of stripping column 9 as an off gas, and are introduced into condenser 14, which is typically operated at a temperature of about -25° C. While under reflux conditions, pump 15 returns a portion of the CFC-115 to the stripping column 9. The remaining portion of the CFC-115 exits the system via conduit 16.

An off gas is also removed from extraction column 2, with the exception that the off gas from column 2 contains HFC-125 product which is substantially free from CFC-115 of the first mixture. The HFC-125 product is transported via conduit 17 to condenser 18. Condenser 18 is typically operated at a temperature of about -25°C. While under reflux conditions, pump 19 returns a portion of the HFC-125 product to extraction column 2. The HFC-125 product exits the system via conduit 20.

The following examples are provided to illustrate certain aspects of the present invention; but not limit the scope of the invention as defined by the appended claims. Parts per million (ppm) concentrations are by weight unless otherwise specified. The following Examples employ the NRTL interaction parameters identified above. All Examples are based upon a feed rate of pentafluoroethane of about 1000 lb/hr, with the impurity content of chloropentafluoroethane at a level of either approximately 5 lb/hr (4975 ppm) or 50 lb/hr (4.8%). A different column design and operating conditions are used for the Examples in which extractive distillation is employed than those in which conventional distillation is employed, in order to enhance the performance of each distillation method.

In the following Comparative Examples involving conventional distillation, the number of stages in the distillation column is either 101 or 121 stages, and the pentafluoroethane stream to be purified is fed onto column stage 50, with the column condenser corresponding to stage no.1. Depending upon the desired product purity, the operating conditions are selected and various reflux ratios tested to maximize the yield of such a product.

### Comparative Example 1

In this comparative example, conventional distillation is used in a column with 121 theoretical stages for purifying a feed stream containing 1000 lb/hr of pentafluoroethane (HFC-125) and 5 lb/hr of chloropentafluoroethane (CFC-115), for a concentration of 4975 ppm. The feed is introduced at a temperature of -25 degrees C, with the column condenser pressure at 194.7 psia and the column base pressure 3 psi higher. The distillate temperature is 23.8 degrees C, and the bottom column temperature is 24.4 degrees C. Under these operating conditions, the product HFC-125 will leave in the overhead (distillate) stream from the column. The remaining HFC-125 exits in the bottoms stream with CFC-115 as a waste product. Conditions are set so that about 50% of the feed goes into the overhead and 50% to the bottoms.

**Table 4**

| Reflux Flow (lb/hr) | Reflux Ratio | Lb/hr HFC-125 in Overhead | ppm CFC-115 in Overhead | % HFC-125 Recovery |
|---|---|---|---|---|
| 100000 | 200/1 | 501 | 2116 | 50 |
| 200000 | 400/1 | 501 | 1936 | 50 |
| 300000 | 600/1 | 501 | 1871 | 50 |

The product purity is relatively low, even with a relatively high reflux ratio of 600/1 and a large number of theoretical separation stages in the column.

### Comparative Example 2

In this comparative example, conventional distillation is used in a column with 101 theoretical stages for purifying a feed stream of the same composition as Comparative Example 1. The operating conditions of the distillation column are modified to employ the CFC-115/HFC-125 azeotrope, discussed above that exists at very low temperatures, by taking the azeotrope overhead as a means of removing the CFC-115 in the feed. The feed is introduced at a temperature of -25 degrees C, with the column condenser pressure set at 19.7 psia and the column base pressure 3 psi higher. Under these conditions the distillate temperature is -42 degrees C, and the bottom column temperature is -38.8 degrees C. Under these operating conditions, the product HFC-125 leaves in the bottom stream from the column. An impurity level of 500 ppm CFC-115 is selected. The remaining HFC-125 is taken overhead with azeotroped CFC-115 as a waste product.

**Table 5**

| Reflux flow (lb/hr) | Reflux Ratio | Lb/hr HFC-125 in Bottoms | ppm CFC-115 in Bottoms | % HFC-125 Recovery |
|---|---|---|---|---|
| 10000 | 21/1 | 529 | 500 | 52.9 |
| 25000 | 329/1 | 930 | 500 | 93.0 |
| 50000 | 862/1 | 950 | 500 | 95.0 |
| 100000 | 1852/1 | 955 | 500 | 95.5 |

At a reflux ratio of 1852/1, and with the impurity level of 500 ppm CFC-115, the yield of HFC-125 product is 95.5%.

### Comparative Example 3

In this comparative example, conditions are the same as Comparative Example 2 except that product purity requirement is increased: that is, the maximum allowed level of CFC-115 impurity is reduced from 500 to 50 ppm.

**Table 6**

| Reflux flow (lb/hr) | Reflux Ratio | Lb/hr HFC-125 in Bottoms | ppm CFC-115 in Bottoms | % HFC-125 Recovery |
|---|---|---|---|---|
| 50000 | 115/1 | 565 | 50 | 56.5 |
| 100000 | 585/1 | 832 | 50 | 83.2 |
| 150000 | 1083/1 | 865 | 50 | 86.5 |

At a reflux ratio of 1083/1, about 15% of the HFC-125 in the feed is lost with the distillate.

The above Examples show that conventional distillation is not an economical way to separate CFC-115 from HFC-125 when high purity HFC-125 is desired because such require a relatively high reflux ratio, a large column diameter to accommodate this high reflux ratio, high utilities cost for heating and cooling this large flow of material through the column, and the need for disposing of large quantities of waste material containing CFC-115. Such waste material will also contain a relatively large quantity of HFC-125.

In the following Examples involving extractive distillation, which are provided to illustrate; but not limit the scope of the invention, the distillation column has only 57 theoretical stages, again with the column condenser counted as stage no. 1. The pentafluoroethane stream to be purified is introduced onto column stage 40, and the extractant is fed onto column stage 15. In each Example, the column distillate/feed ratio (based on pentafluoroethane) and the reflux ratio are set to achieve the desired product purity and recovery (yield), and operating temperatures and pressures are varied as necessary to maximize operating conditions. Except for Example 2, column pressures are selected so that the bottoms temperature is approximately 100 degrees C. Each of the Examples also includes a measurement of performance at varied levels of extractant flow rates.

### Example 1

In this example, extractive distillation with normal pentane is used in a column with 57 theoretical stages for purifying a feed stream containing 1000 lb/hr of pentafluoroethane (HFC-125) and 5 lb/hr of chloropentafluoroethane (CFC-115) or 4975 ppm as in the Comparative Examples. The feed and extractant are introduced at a temperature of -5 degrees C, with the column condenser pressure set at 84.7 psia and the column base pressure 3 psi higher. The normal pentane is fed at varying rates as shown in the table, while the reflux ratio is held at only 3/1. The distillate temperature is -4.4 degrees C, and the bottom column temperature is about 100 degrees C. The pentane and most of the CFC-115 leave the bottom of the column, and the product HFC-125 leaves the top of the column as overhead.

**Table 7**

| Lb/hr Pentane Feed Rate | Lb/hr HFC-125 in Overhead | ppm CFC-115 in Overhead | ppm Extractant in Overhead | % HFC-125 Recovery |
|---|---|---|---|---|
| 2500 | 999 | 911 | 5.8 | 99.9 |
| 5000 | 999 | 73 | 6.1 | 99.9 |
| 7500 | 999 | 12 | 6.3 | 99.9 |
| 10000 | 999 | 3.3 | 6.5 | 99.9 |

### Example 2

Example 2 is carried out in substantially the same way as Example 1 except that the column condenser pressure is 34.7 psia instead of 84.7 psia, and the feed and pentane are introduced at a temperature of -25 degrees C, with the column condenser pressure set at 34.7 psia and the column base pressure 3 psi higher. The distillate temperature is -29 degrees C, and the bottom column temperature is about 66 degrees C.

**Table 8**

| Lb/hr Pentane Feed Rate | Lb/hr HFC-125 in Overhead | ppm CFC-115 in Overhead | ppm Extractant in Overhead | % HFC-125 Recovery |
|---|---|---|---|---|
| 2500 | 999 | 1720 | <0.1 | 99.9 |
| 5000 | 999 | 148 | <0.1 | 99.9 |
| 7500 | 999 | 21 | <0.1 | 99.9 |
| 10000 | 999 | 5.0 | < 0.1 | 99.9 |

In both Examples 1 and 2, extractive distillation with pentane permits a relatively high purity HFC-125 product to be obtained at high product yield, while using a relatively small distillation column and reflux ratio.

### Example 3

Example 3 is carried out in substantially the same way as Example 1 except that the impure HFC-125 feed contains ten times as much CFC-115, i.e. a flow rate of 50 lb/hr instead of 5 lb/hr, for a concentration of 4.8%. The distillate temperature is -4.4 degrees C, and the bottom column temperature ranges from 95 to 100 degrees C, depending on the flow rate of pentane.

**Table 9**

| Lb/hr Pentane Feed Rate | Lb/hr HFC-125 in Overhead | ppm CFC-115 in Overhead | ppm Extractant in Overhead | % HFC-125 Recovery |
|---|---|---|---|---|
| 2500 | 999 | 4175 | 5.6 | 99.9 |
| 5000 | 999 | 431 | 6.1 | 99.9 |
| 7500 | 999 | 81 | 6.3 | 99.9 |
| 10000 | 999 | 24 | 6.4 | 99.9 |
| 15000 | 999 | 4.9 | 6.6 | 99.9 |
| 20000 | 999 | 1.8 | 6.8 | 99.9 |

This demonstrates that using pentane as an extractive agent permits obtaining high purity HFC-125 from a mixture having an impurity concentration of about ten times greater than in the Comparative Examples.

### Example 4

Example 4 is carried out in substantially the same way as Example 1 except that the extractant is cyclopentane and the column pressure is 54.7 psia. The distillate temperature is -17 degrees C, and the bottom column temperature ranges from 94 to 98 degrees C, depending on the flow rate of extractant.

**Table 10**

| Lb/hr Extract. Feed Rate | Lb/hr HFC-125 in Overhead | ppm CFC-115 in Overhead | ppm Extractant in Overhead | % HFC-125 Recovery |
|---|---|---|---|---|
| 1000 | 999 | 918 | <0.01 | 99.9 |
| 1500 | 999 | 168 | <0.01 | 99.9 |
| 2500 | 999 | 11 | <0.01 | 99.9 |
| 5000 | 999 | 0.2 | <0.01 | 99.9 |
| 7500 | 999 | <0.1 | <0.01 | 99.9 |

### Example 5

Example 5 is carried out in substantially the same way as Example 2 except that the extractant is normal heptane, the column condenser pressure is 12.7 psia, and the feed and extractant are introduced at a temperature of -40 degrees C. The distillate temperature is -51 degrees C, and the bottom column temperature ranges from 97 to 100 degrees C, depending on the flow rate of extractant.

**Table 11**

| Lb/hr Heptane Feed Rate | Lb/hr HFC-125 in Overhead | ppm CFC-115 in Overhead | ppm Extractant in Overhead | % HFC-125 Recovery |
|---|---|---|---|---|
| 2500 | 999 | 307 | < 0.01 | 99.9 |
| 5000 | 999 | 6.9 | <0.01 | 99.9 |
| 7500 | 999 | 0.67 | <0.01 | 99.9 |
| 10000 | 999 | 0.14 | < 0.01 | 99.9 |

### Example 6

Example 6 is carried out in substantially the same way as Example 2 except that the extractant is perchloroethylene (perclene), the column condenser pressure is 4.7 psia, and the feed and extractant are introduced at a temperature of 40 degrees C. The distillate temperature is -69 degrees C, and the bottom column temperature ranges from 93 to 98 degrees C, depending on the flow rate of extractant.

**Table 12**

| Lb/hr Perclene Feed Rate | Lb/hr HFC-125 in Overhead | ppm CFC-115 in Overhead | ppm Extractant in Overhead | % HFC-125 Recovery |
|---|---|---|---|---|
| 5000 | 999 | 3586 | <0.01 | 99.9 |
| 10000 | 999 | 695 | <0.01 | 99.9 |
| 20000 | 999 | 436 | <0.01 | 99.9 |
| 30000 | 999 | 533 | < 0.01 | 99.9 |
| 40000 | 999 | 411 | <0.01 | 99.9 |
| 50000 | 999 | 245 | <0.01 | 99.9 |

### Example 7

Example 7 is carried out in substantially the same way as Example 2 except that the extractant is trichloroethylene (triclene), the column condenser of pressure is 18.7 psia, and the feed and extractant are introduced at a temperature of 40 degrees C. The distillate temperature is -43 degrees C, and the bottom column temperature ranges from 99 to 100 degrees C, depending on the flow rate of extractant.

**Table 13**

| Lb/hr Triclene Feed Rate | Lb/hr HFC-125 in Overhead | ppm CFC-115 in Overhead | ppm Extractant in Overhead | % HFC-125 Recovery |
|---|---|---|---|---|
| 5000 | 999 | 4680 | <0.01 | 99.9 |
| 10000 | 999 | 3014 | <0.01 | 99.9 |
| 20000 | 999 | 782 | <0.01 | 99.9 |
| 30000 | 999 | 251 | <0.01 | 99.9 |
| 40000 | 999 | 106 | <0.01 | 99.9 |
| 50000 | 999 | 55 | <0.01 | 99.9 |

### Example 8

Example 8 is carried out in substantially the same way as Example 1 except that the extractant is a mixture of 50% by weight normal pentane and 50% hexane, the column condenser pressure is 64.7 psia, and the feed and extractant are introduced at a temperature of -10 degrees C. The distillate temperature is -12 degrees C, and the bottom column temperature ranges from 103 to 104 degrees C, depending on the flow rate of extractant.

**Table 14**

| Lb/hr Extract. Feed Rate | Lb/hr HFC-125 in Overhead | ppm CFC-115 in Overhead | ppm Extractant in Overhead | % HFC-125 Recovery |
|---|---|---|---|---|
| 2500 | 999 | 200 | 0.8 | 99.9 |
| 5000 | 999 | 7.8 | 0.9 | 99.9 |
| 7500 | 999 | 1.1 | 0.9 | 99.9 |
| 10000 | 999 | 03 | 1.0 | 99.9 |

This Example illustrates that a mixed extractant may be effectively used for in the inventive process. In some cases, such mixtures may be available at a much lower price than pure materials. For example, by using a mixture of extractant agents the inventive process can be maximized to obtain HFC-125 that has the desired purity while minimizing the extractive agent cost. Alternatively, a series of one or more chemically similar or distinct extractive agents can be employed for maximizing the effectiveness of the inventive process.

## Claims

1. A process for separating chloropentachloroethane from a first mixture comprising chloropentafluoroethane and pentafluoroethane, the process comprising the steps of:
adding at least one extractive agent to the first mixture in order to form a second mixture, wherein the extractive agent is fluorine free and does not hydrogen bond; and wherein the extractive agent has an atmospheric boiling point greater than 28 degrees C, but excluding the use of an extractive agent consisting of n-hexane, diethylether, ethyl acetate, acetone or methylethyl ketone,
separating the chloropentafluoroethane from the pentafluoroethane of the second mixture by extractively distilling the second mixture in an extractive distillation zone, and;
recovering pentafluoroethane.

2. The process of claim 1 wherein the extractive agent has at least two carbon atoms.

3. The process of claim 2 wherein the extractive agent is at least one of a hydrocarbon, chlorocarbon and hydrochlorocarbon and wherein the hydrocarbon extractant agent has at least five carbon atoms and the chlorocarbon or hydrochlorocarbon extractant has at least two carbon atoms.

4. The process of claim 3 wherein the extractive agent comprises at least one hydrocarbon containing 5 to 8 carbons, their isomers and cyclic compounds thereof having an atmospheric boiling point greater than 28 degrees C and less than 126 degrees C.

5. The process of claim 3 wherein the extractive agent comprises at least one chlorocarbon and hydrochlorocarbon containing 2 carbon atoms, their isomers and cyclic compounds having an atmospheric boiling point greater than 28 degrees C and less than 126 degrees C.

6. The process of claim 3 wherein the extractive agent comprises at least one member selected from the group consisting of perchloroethylene, methyl chloroform, 1,1, dichloroethane, 1,2 dichloroethane, trichloroethylene, and ethylene dichloride.

7. The process of claim 3 wherein the extractive agent comprises at least one member selected from the group consisting of n-pentane, heptane, octane, their isomers and cyclic compounds thereof having an atmospheric boiling point greater than 28 degrees C and less than 126 degrees C.

## Patentansprüche

1. Verfahren zur Abtrennung von Chlorpentafluorethan aus einer ersten Mischung, die Chlorpentafluorethan und Pentafluorethan umfaßt, umfassend die folgenden Stufen:
Zugabe mindestens eines Extraktionsrnittels zur ersten Mischung, um eine zweite Mischung zu bilden, wobei das Extraktionsmittel Fluor-frei ist und keine Wasserstoffbrückenbindungen ausbildet; und wobei das Extraktionsmittel einen Atmosphären-Siedepunkt von höher als 28° C aufweist, aber unter Ausschluß der Verwendung eines Extraktionsmittels, das aus n-Hexan, Diethylether, Ethylacetat, Aceton oder Methylethylketon besteht,
Abtrennung des Chlorpentafluorethans von dem Pentafluorethan der zweiten Mischung durch extraktives Destillieren der zweiten Mischung in einer Extraktionsdestillationszone und
Isolierung von Pentafluorethan.

2. Verfahren nach Anspruch 1, in welchem das Extraktionsmittel mindestens 2 Kohlenstoffatome aufweist.

3. Verfahren nach Anspruch 2, in welchem das Extraktionsmittel mindestens eines von einem Kohlenwasserstoff, Chlorkohlenstoff und Chlorkohlenwasserstoff ist und worin das Kohlenwasserstoff-Extraktionsmittel mindestens fünf Kohlenstoffatome aufweist und das Chlorkohlenstoff- oder Chlorkohlenwasserstoff-Extraktionsmittel mindestens 2 Kohlenstoffatome aufweist.

4. Verfahren nach Anspruch 3, in welchem das Extraktionsmittel mindestens einen fünf bis acht Kohlenstoffe enthaltenden Kohlenwasserstoff, dessen Isomere und zyklische Verbindungen davon mit einem Atmosphären-Siedepunkt von höher als 28° C und niedriger als 126° C umfaßt.

5. Verfahren nach Anspruch 3, in welchem das Extraktionsmittel mindestens einen Chlorkohlenstoff und Chlorkohlenwasserstoff, der zwei Kohlenstoffatome enthält, deren Isomere und zyklische Verbindungen mit einem Atmosphären-Siedepunkt von höher als 28° C und niedriger als 126° C umfaßt.

6. Verfahren nach Anspruch 3, in welchem das Extraktionsmittel mindestens ein Mitglied umfaßt, das aus der aus Perchlorethylen, Methylchloroform, 1,1-Dichlorethan, 1,2-Dichlorethan, Trichlorethylen und Ethylendichlorid bestehenden Gruppe ausgewählt ist.

7. Verfahren nach Anspruch 3, in welchem das Extraktionsmittel mindestens ein Mitglied umfaßt, das aus der aus n-Pentan, Heptan, Oktan, deren Isomeren und zyklischen Verbindungen davon mit einem Atmosphären-Siedepunkt von höher als 28° C und niedriger als 126° C bestehenden Gruppe ausgewählt ist.

## Revendications

1. Procédé de séparation de chloropentafluoroéthane à partir d'un premier mélange composé de chloropentafluoroéthane et de pentafluoroéthane, le procédé comprenant les étapes consistant à :
ajouter au moins un agent d'extraction au premier mélange afin de former un deuxième mélange, l'agent d'extraction étant exempt de fluor et ne formant pas de liaison hydrogène, et l'agent d'extraction ayant un point d'ébullition atmosphérique supérieur à 28°C, mais en excluant l'utilisation d'un agent d'extraction composé de n-hexane, de diéthyléther, d'acétate d'éthyle, d'acétone ou de méthyléthylcétone;
séparer le chloropentafluoroéthane du pentafluoroéthane du deuxième mélange par distillation extractive du deuxième mélange dans une zone de distillation extractive, et
récupérer le pentafluoroéthane.

2. Procédé suivant la revendication 1, dans lequel l'agent d'extraction a au moins deux atomes de carbone.

3. Procédé suivant la revendication 2, dans lequel l'agent d'extraction est au moins l'un d'un hydrocarbure, d'un chlorocarbone et d'un hydrogénochlorocarbone, et dans lequel l'agent d'extraction hydrocarboné a au moins cinq atomes de carbone et l'agent d'extraction chlorocarboné ou hydrogénochlorocarboné a au moins deux atomes de carbone.

4. Procédé suivant la revendication 3, dans lequel, l'agent d'extraction comprend au moins un hydrocarbure contenant 5 à 8 atomes de carbone, leurs isomères et les composés cycliques de ceux-ci ayant un point d'ébullition atmosphérique supérieur à 28°C et inférieur à 126°C.

5. Procédé suivant la revendication 3, dans lequel l'agent d'extraction comprend au moins un chlorocarbone et un hydrogénochlorocarbone contenant 2 atomes de carbone, leurs isomères et composés cycliques ayant un point d'ébullition atmosphérique supérieur à 28°C et inférieur à 126°C.

6. Procédé suivant la revendication 3, dans lequel l'agent d'extraction comprend au moins un membre sélectionné dans le groupe composé du perchloroéthylène, du méthylchloroforme, du 1,1-dichloroéthane, du 1,2-dichloroéthane, du trichloroéthylène et du dichlorure d'éthylène.

7. Procédé suivant la revendication 3, dans lequel l'agent d'extraction comprend au moins un membre sélectionné dans le groupe composé du n-pentane, de l'heptane, de l'octane, de leurs isomères et des composés cycliques de ceux-ci ayant un point d'ébullition atmosphérique supérieur à 26°C et inférieur à 126°C.
